(19) Europäisches Patentamt
European Patent Office
Office européen des brevets

(11) EP 0 695 740 B1

(12) EUROPEAN PATENT SPECIFICATION

(45) Date of publication and mention of the grant of the patent:
**22.11.2000 Bulletin 2000/47**

(51) Int. Cl.[7]: **C07C 317/14**, G03F 7/22, C07C 39/16

(21) Application number: **95111852.0**

(22) Date of filing: **27.07.1995**

(54) **Quinonediazide sulfonic acid esters and positive photoresist compositions comprising the same**

Sulfonsäurechinondiazidester und diese enthaltende positiv-arbeitende Fotolackzusammensetzungen

Esters d'acide sulfoniques de quinolinediazide et compositions pour une photoréserve positive les contenant

(84) Designated Contracting States:
**DE FR GB IT**

(30) Priority: **05.08.1994 JP 18462794**
**17.03.1995 JP 5882695**
**17.03.1995 JP 5882795**

(43) Date of publication of application:
**07.02.1996 Bulletin 1996/06**

(73) Proprietor:
**SUMITOMO CHEMICAL COMPANY LIMITED**
**Osaka-shi, Osaka 541-0041 (JP)**

(72) Inventors:
- **Sakamoto, Hiromu**
  **Minoo-shi, Osaka (JP)**
- **Osaki, Haruyoshi**
  **Toyonaka-shi, Osaka (JP)**
- **Ichikawa, Koji**
  **Tondabayashi-shi, Osaka (JP)**
- **Uetani, Yasunori**
  **Toyonaka-shi, Osaka (JP)**

(74) Representative:
**VOSSIUS & PARTNER**
**Siebertstrasse 4**
**81675 München (DE)**

(56) References cited:
**US-A- 5 173 389**

- **PATENT ABSTRACTS OF JAPAN vol. 18 no. 284 (P-1745) ,30 May 1994 & JP-A-06 051507 (JAPAN SYNTHETIC RUBBER CO. LTD.) 25 February 1994**
- **CHEMICAL ABSTRACTS, vol. 115, no. 16, 21 October 1991 Columbus, Ohio, US; abstract no. 170720v, TZENG ET AL 'DQN photoresist with tetrahydroxydiphenylmethane as ballasting group in photoactive compound' page 800; column 1; & PROC.SPIE-INT. SOC.OPT., 1991 pages 469-476,**
- **CHEMICAL ABSTRACTS, vol. 115, no. 12, 23 September 1991 Columbus, Ohio, US; abstract no. 123675z, TZENG ET AL 'DQN photoresrist with tetrahydroxydiphenylmethane as ballasting group in PAC.' page 622; column 2; & MRL BULL.RES. DEV, vol. 5, no. 1, 1991 pages 23-7,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention relates to a quinonediazide sulfonic acid ester of a polyhydric phenol compound, a process for producing the same, a sensitizer containing the same and a positive photoresist composition comprising the same.

The present invention also relates to a polyhydric phenol compound which is useful as an ingredient of a positive resist composition, a process for producing the same and a resist composition comprising the same.

Various quinonediazide sulfonic acid esters of compounds having a phenolic hydroxy group have been proposed as sensitizers in photosensitive resin compositions used in minute processes for producing semiconductor. Recently, with a rise in the integration level of integrated circuits, more and more minute processes and formation of pattern of submicron order have been required for producing a semiconductor. In order to cope with the recent tendency, a positive resist composition free from scum (undeveloped residue) and excellent in properties such as sensitivity, resolution, γ-value, profile (developed pattern shape) and heat resistance, etc. is demanded. Particularly in the production of 16-64 MDRAMs (Mega-bit dynamic random access memory), a positive resist composition excellent in the balance among properties such as sensitivity, resolution and profile, etc. is demanded. However, for example, sensitivity and resolution have contrary tendency, i.e. improving the sensitivity often lowers the resolution, and vice versa. Therefore, it is generally difficult to obtain a positive resist composition excellent in both properties.

In USP-5,173,389 (=JP-A-2-285351) and USP-5,290,656(=JP-A-2-269351), there is proposed a positive photoresist which comprises, as a photosensitizer, a quinonediazide sulfonic acid ester of a polyhydric phenol compound having two or more benzene rings. The positive photoresist, however, is not yet satisfactory in properties, such as sensitivity, resolution, γ-value, profile, scum, etc., particularly in sensitivity and in compatibility between sensitivity and resolution.

An object of the present invention is to provide a photosensitive compound which is useful in producing a positive resist composition excellent in the balance among properties, such as sensitivity, resolution, γ-value, profile and heat resistance, and free from scum; a process for preparing the same, a photosensitizer comprising the same and a positive resist composition comprising the same.

This object could be achieved on the basis of the finding that an o-quinonediazide sulfonic acid ester of a compound having a specific molecular structure and having four phenolic hydroxy groups can give excellent properties to a positive resist composition when it is used as the sensitizer in the positive resist composition.

Chemical Abstract 115:170720v and Chemical Abstract 115:123675z describe a photoactive compound for a resist system which is an esterification product of 1,2-naphthoquinone diazide-5-sulphonyl chloride and 2,3,4,4'-tetrahydroxydiphenylmethane.

JP-A-6051507 describes positive resists containing an alkali-soluble novolak resin and an 1,2-naphthoquinonediazido-4-sulfonic ester as a photosensitive component.

A further object of the present invention is to provide a novel polyhydric phenol compound which is useful as an ingredient of a resist composition, a process for producing the same in high purity through an easy reaction, and a resist composition comprising the same.

Hitherto, various polyhydric phenols have been known. For example, in USP-4,514,334(=JP-A-55-162728) and USP-4,894,432(=JP-A-62-84035), there is described a polyhydric phenol having two or more benzene rings. However, in order to produce the known polyhydric phenol compounds, long producing processes are required. According to the processes, often a compound of low purity is obtained and it is difficult to produce a compound of high purity by refining the compound of low purity.

A quinonediazide sulfonic acid ester of a polyhydric phenol compound has been proposed as a photosensitizer comprised in a resist composition used in minute producing processes of semiconductors, as mentioned above and in various literature such as EP-A-573,056(=JP-A-6-167805). A polyhydric phenol compound can also be used as an alkali soluble ingredient in a resist composition, as mentioned in JP-A-2-2560 or JP-A-3-200253.

The above object could be achieved on the basis of a novel aromatic tetrahydroxy compound which is easily obtained in high purity and is useful as an ingredient of a resist composition.

The present invention relates to the subject-matter disclosed in the claims.

The present invention provides a compound represented by the general formula (I):

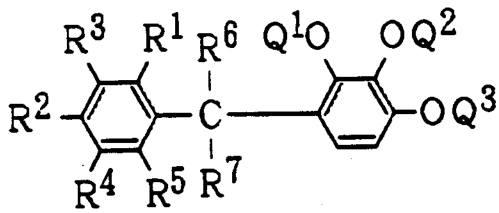

(I)

wherein $R^2$ represents $-OQ^4$ and $R^1$, $R^3$, $R^4$ and $R^5$ each independently represent hydrogen or alkyl having 6 or less carbon atoms, provided that at least one of $R^1$ and $R^5$ is alkyl having 6 or less carbon atoms;

> $R^6$ and $R^7$ each independently represent hydrogen, alkyl having 6 or less carbon atoms or alkenyl having 6 or less carbon atoms, or $R^6$ and $R^7$ form a cycloalkane ring having 6 or less carbon atoms together with the carbon atom to which they are attached; and
> $Q^1$, $Q^2$, $Q^3$ and $Q^4$ each independently represent hydrogen or an o-quinonediazide sulfonyl group, provided that at least one of $Q^1$, $Q^2$, $Q^3$ and $Q^4$ is an o-quinonediazide sulfonyl group.

The present invention also provides a process for producing the compound of formula (I), which comprises allowing a tetrahydroxy compound represented by the general formula (II):

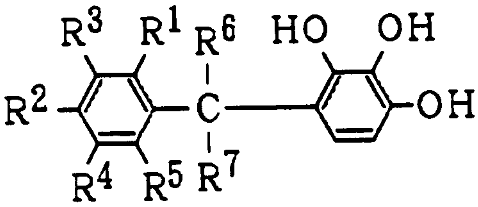

(II)

wherein $R^2$ represents hydroxy; $R^1$, $R^3$, $R^4$ and $R^5$ each independently represent hydrogen or alkyl having 6 or less carbon atoms, provided that at least one of $R^1$ and $R^5$ is alkyl having 6 or less carbon atoms; and

> $R^6$ and $R^7$ each independently represent hydrogen, alkyl having 6 or less carbon atoms or alkenyl having 6 or less carbon atoms, or $R^6$ and $R^7$ form a cycloalkane ring having 6 or less carbon atoms together with the carbon atom to which they are attached to react with an o-quinonediazide sulfonyl halide in the presence of a base.

The present invention also provides a photosensitizer which comprises the compounds of formula (I) as its effective ingredient.
The present invention also provides a positive photoresist composition which comprises the compounds of formula (I) and an alkali soluble novolac resin.
The present invention further provides a novel compound, 1,2,3-trihydroxy-4-(4-hydroxy-2,5-dimethylbenzyl) benzene, which is one of compounds represented by the formula (II), more concretely represented by the following formula (A):

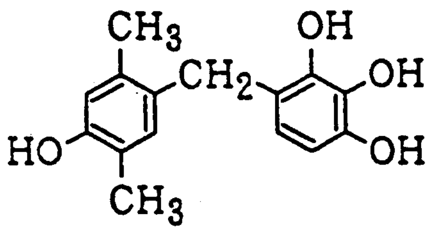

(A)

and is useful as an ingredient of a resist composition. (Hereinafter, the compound of formula (A) is described as "compound (A)".)

The present invention still further provides a process for producing compound (A) which comprises allowing 4-hydroxymethyl-2,5-dimethylphenol to react with pyrogallol, and provides a resist composition comprising compound (A).

Alkyl denoted by $R^1$, $R^3$, $R^4$ or $R^5$ in the formula (I) or (II) is preferably a straight chain or branched chain alkyl group having 4 or less carbon atoms. Alkyl denoted by $R^6$ or $R^7$ is preferably methyl or ethyl. Examples of o-quinonediazide sulfonyl groups denoted by $Q^1$, $Q^2$, $Q^3$ and $Q^4$ in the formula (I) include the 1,2-naphthoquinonediazide-4-sulfonyl, 1,2-naphthoquinonediazide-5-sulfonyl and 1,2-benzoquinonediazide-4-sulfonyl group, which are represented by the following formulae, respectively:

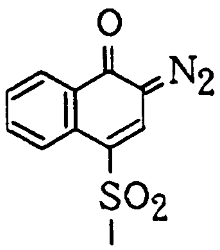 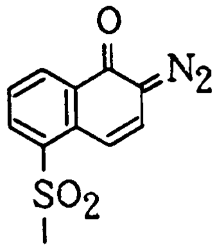 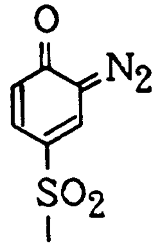

Among the compounds of formula (I), wherein $R^2$ is $-OQ^4$, examples of the particularly important compounds include:

(1) the compounds of formula (I) wherein one of $R^3$ and $R^4$ is hydrogen and the other is alkyl, and $R^1$ and $R^5$ are each independently hydrogen or alkyl;
(2) the compounds of formula (I) wherein $R^3$ and $R^4$ are each independently alkyl, and one of $R^1$ and $R^5$ is hydrogen and the other is alkyl; and
(3) the compounds of formula (I) wherein both of $R^3$ and $R^5$ are hydrogen, and both of $R^1$ and $R^4$ is methyl.

Tetrahydroxy compounds of formula (II) wherein $R^6$ and $R^7$ are hydrogen can be produced by allowing a 4-hydroxybenzyl alcohol, which may be further substituted at the benzene ring, to react with pyrogallol.

Tetrahydroxy compound of formula (II) wherein at least one of $R^6$ and $R^7$ is alkyl can be produced by allowing a 4-alkenylphenol which has a double bond at the $\alpha$-position and may be further substituted at the benzene ring to react with pyrogallol. For example, tetrahydroxy compounds of formula (II) wherein $R^6$ and $R^7$ are methyl can be produced by allowing 4-isopropenyl phenol, which may be further substituted at the benzene ring, to react with pyrogallol.

Tetrahydroxy compound of formula (II) wherein at least one of $R^6$ and $R^7$ is alkyl can also be produced by allowing a 4-hydroxybenzyl alcohol which is substituted by alkyl at the $\alpha$-position and may be further substituted at the benzene ring, to react with pyrogallol.

The above-mentioned reaction to produce a tetrahydroxy compound of formula (II) is preferably carried out in the presence of an acid catalyst to accelerate the reaction. As the acid catalyst, either inorganic acids or organic acid can be used. Examples of the acid catalyst include hydrochloric acid, sulfuric acid, formic acid, oxalic acid and p-toluenesulfonic acid.

As examples of tetrahydroxy compounds of formula (II) usable as a raw material for producing compounds of formula (I), following compounds can be mentioned:

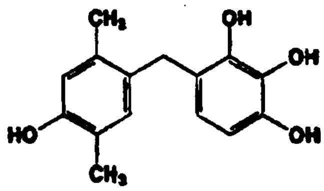

(b)

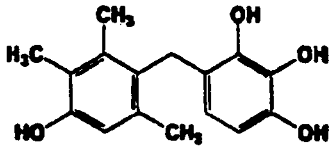

(c)

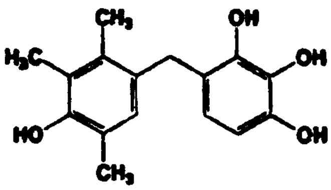

(d)

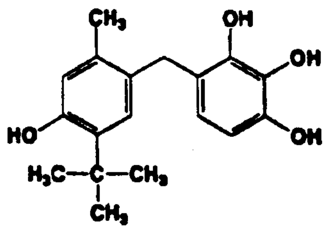

(e)

Compound (A) and above-mentioned compound (b) are the same compounds. Compound (A) can be easily produced in high purity by allowing 4-hydroxymethyl-2,5-dimethylphenol to react with pyrogallol. In the reaction, the amount of pyrogallol is preferably 1-3 moles, more preferably 1-2 moles per 1 mole of 4-hydroxymethyl-2,5-dimethylphenol. Though any of the acid catalysts mentioned above as those usable for producing tetrahydroxy compound of formula (II) can be used in this reaction, hydrochloric acid, sulfuric acid and p-toluenesulfonic acid are preferred and among them, p-toluenesulfonic acid is most preferred. The amount of the acid catalyst is usually 0.01-0.1 mole, preferably 0.01-0.05 mole, more preferably 0.01-0.03 moles per 1 mole of pyrogallol.

The reaction to produce compound (A) is usually carried out in the presence of a solvent, and water is preferably used as the solvent. The amount of solvent, particularly water, is usually 0.25-5 parts by weight, preferably 0.5-2 parts by weight, more preferably 0.8-1.2 parts by weight based on 1 part by weight of pyrogallol.

The reaction to produce compound (A) is usually carried out under atmospheric pressure at a temperature of about 10-60°C. As long as the raw materials can be dissolved in the reaction system, the lower reaction temperature is more preferred. 4-Hydroxymethyl-2,5-dimethylphenol is preferably added to an aqueous solution containing pyrogallol and an acid catalyst slowly or dividedly, for example over 0.5-2 hours.

When water is used as the solvent, compound (A) precipitates as the reaction is proceeding or by cooling the reaction system to room temperature after the completion of the reaction. Then, solid-liquid separation is carried out to obtain crude compound (A), which is preferably refined. Though the refining method is not limited, crystallization is preferred. As the crystallization, crystallization from a solution in an aromatic solvent, such as benzene, toluene and xylene, and crystallization from an aqueous solution containing alcohol, such as methanol and ethanol can be mentioned. Among the aromatic solvents, toluene is preferably used. Among the aqueous solutions containing alcohol, those containing 30-60 % by weight of alcohol are preferred, those containing 30-40 % by weight of alcohol are more preferred and particularly preferred are those containing methanol.

The esterification reaction of the tetrahydroxy compound of formula (II) with the o-naphthoquinone sulfonyl halide is carried out in the presence of a base. Examples of the base useable in the reaction include amines, such as trimethylamine, triethylamine, triethanolamine, N,N-dimethylaniline, N,N-diethylaniline and pyridine, and inorganic

bases such as sodium carbonate and sodium acid carbonate. The amount of the base is usually 1.05-5 moles, preferably 1.05-1.2 moles, and more preferably 1.1-1.2 moles, per 1 mole of o-naphthoquinone sulfonyl halide.

The esterification reaction is usually carried out in a reaction solvent. As examples of the reaction solvent, ethers, such as 1,4-dioxane and tetrahydrofuran; esters such as amyl acetate and $\gamma$-butyrolactone; and aliphatic ketones, such as acetone and 2-heptanone can be mentioned. These solvents can be used singly or as a mixture of two or more thereof. Among the above-mentioned solvents, 1,4-dioxane is particularly preferred. The amount of the reaction solvent is usually 2-6 parts by weight, preferably 3-5 parts by weight, more preferably 4-5 parts by weight based on 1 part by total weight of tetrahydroxy compound and o-naphthoquinone sulfonyl halide.

After completion of the esterification reaction, the reaction system is neutralized with an acid such as acetic acid and is filtered to remove solids. The filtrate thus obtained is then mixed with dilute aqueous acid solution, such as aqueous acetic acid solution having a concentration of about 1% by weight, to precipitate the objective compound. The precipitate is filtered, washed with water and dried to obtain the compound of formula (I).

The compound of formula (I) can be used as a photosensitizer. The photosensitizer may be a mixture of two or more of;

a monoester which means a compound of formula (I) wherein one of $Q^1$, $Q^2$, $Q^3$ and $Q^4$ is an o-quinonediazide sulfonyl group;
a diester which means a compound of formula (I) wherein two of $Q^1$, $Q^2$, $Q^3$ and $Q^4$ are o-quinonediazide sulfonyl groups;
a triester which means a compound of formula (I) wherein three of $Q^1$, $Q^2$, $Q^3$ and $Q^4$ are o-quinonediazide sulfonylgroups; and
a tetraester which means a compound of formula (I) wherein four(=all) of $Q^1$, $Q^2$, $Q^3$ and $Q^4$ are o-quinonediazide sulfonyl groups.

The amount of o-quinonediazide sulfonyl halide varies depending on the esterification ratio of the compound of formula (I) to be produced. However, it is usually 1.2-4.5 moles, preferably 2-4.2 moles, per 1 mole of the tetrahydroxy compound. The average esterification ratio of the compound of formula (I) is preferably 50% or more. For a photosensitizer, the tetraester is preferred. Particularly preferred is a compound of formula (I) wherein $R^2$ is $-OQ^4$, all of $R^3$, $R^5$, $R^6$ and $R^7$ are hydrogen, both of $R^1$ and $R^4$ are methyl, and all of $Q^1$, $Q^2$, $Q^3$ and $Q^4$ are 1,2-naphthoquinonediazide-5-sulfonyl groups, i.e. 2',5'-dimethyl diphenylmethan-2,3,4,4'-tetrayl tetrakis(6-diazo-5,6-dihydro-5-oxo-1-naphthalene sulfonate).

The tetraester can be produced by allowing a tetrahydroxy compound of formula (II), such as 1,2,3-trihydroxy-4-(4-hydroxy-2,5-dimethylbenzyl)benzene, to react with an o-quinonediazide sulfonyl halide, such as 1,2-naphthoquinonediazide-5-sulfonyl chloride in an amount of about 4-4.2 moles per 1 mole of the tetrahydroxy compound under the conditions mentioned above. Even when the amount of o-naphthoquinonediazide sulfonyl halide is less than 4 moles per 1 mole of the tetrahydroxy compound, usually, a mixture containing tetraester can be produced.

The compound of formula (I) is preferably used as a photosensitizer which is sensitive to radiation such as ultraviolet rays having wave-length of 300-500 nm and far ultraviolet rays including excimer laser. The photosensitizer exhibits excellent properties particularly when used in a positive resist composition together with an alkali soluble novolac resin.

A positive resist composition comprising the compound of formula (I) and an alkali soluble novolac resin may further comprise an o-quinonediazide sulfonic acid ester of an aromatic hydroxy compound other than the tetrahydroxy compound of formula (II). The o-quinonediazide sulfonic acid ester of an aromatic hydroxy compound other than the tetrahydroxy compound of formula (II) which has at least one, preferably two or more, phenolic hydroxy group can be produced by an esterification reaction of the aromatic hydroxy compound with an o-quinonediazide sulfonyl halide. Examples of the aromatic hydroxy compound other than the tetrahydroxy compound of formula (II) include:

an aromatic phenol compound represented by the following formula (III):

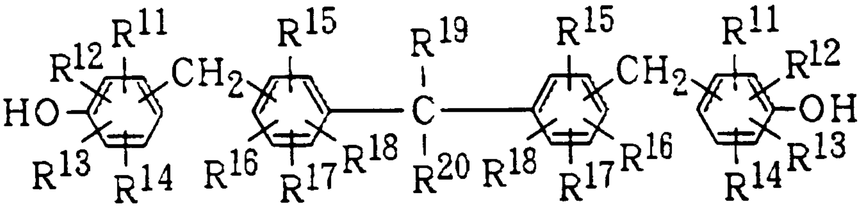

(III)

wherein $R^{11}$, $R^{12}$, $R^{13}$, $R^{14}$, $R^{15}$, $R^{16}$, $R^{17}$ and $R^{18}$ each independently represent hydrogen, hydroxy, alkyl having 6 or less carbon atoms, cycloalkyl having 6 or less carbon atoms or alkoxy having 6 or less carbon atoms, and $R^{19}$ and $R^{20}$ each independently represent hydrogen, alkyl having 6 or less carbon atoms, alkoxy having 6 or less carbon atoms, cycloalkyl having 6 or less carbon atoms or phenyl which may be optionally substituted by one or more substituents selected from hydroxy, alkyl having 6 or less carbon atoms, alkoxy having 6 or less carbon atoms and cycloalkyl having 6 or less carbon atoms;

an aromatic phenol compound represented by the following formula (IV):

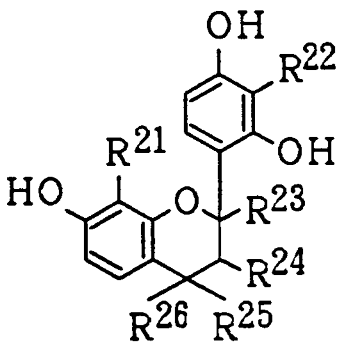

(IV)

wherein $R^{21}$ and $R^{22}$ each independently represent hydrogen, hydroxy or alkyl having 6 or less carbon atoms, and $R^{23}$, $R^{24}$, $R^{25}$ and $R^{26}$ each independently represent hydrogen or alkyl having 6 or less carbon atoms, provided that the ends of $R^{23}$ and $R^{24}$ may be combined to form a cycloalkane ring having 6 or less carbon atoms together with the carbon atoms to which they are attached and/or the ends of $R^{25}$ and $R^{26}$ may be combined to form a cycloalkane ring having 6 or less carbon atoms together with the carbon atom to which they are attached;
an aromatic phenol compound represented by the following formula (V):

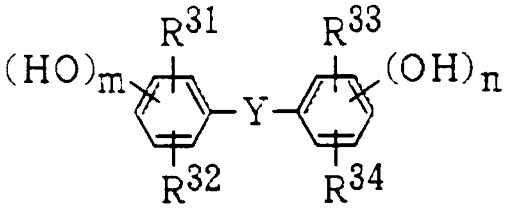

(V)

wherein $R^{31}$, $R^{32}$, $R^{33}$ and $R^{34}$ each independently represent hydrogen or alkyl having 6 or less carbon atoms, m and n each independently represent 0 or a positive integer provided that $m+n \geqq 2$, and Y represents $-C(R^{35})(R^{36})-$ or $-C(R^{37})(R^{38})-Ar-C(R^{39})(R^{40})-$ wherein $R^{35}$, $R^{36}$, $R^{37}$, $R^{38}$, $R^{39}$, and $R^{40}$ each independently represent hydrogen, alkyl having 6 or less carbon atoms or cycloalkyl having 6 or less carbon atoms and Ar represents a divalent hydroxy benzene residue which may be substituted by alkyl having 6 or less carbon atoms; and
an aromatic phenol compound represented by the following formula (VI):

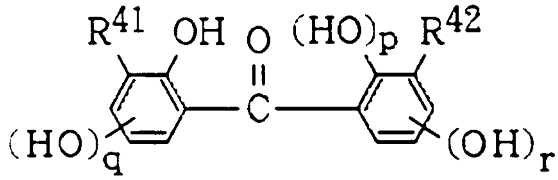

(VI)

wherein $R^{41}$ and $R^{42}$ each independently represent alkyl having 6 or less carbon atoms, aryl having 10 or less carbon atoms, p represents 0 or 1, and q and r each independently represent an integer of 0-3 provided that $q+r \geqq 2$.

The o-quinonediazide sulfonic acid ester of the aromatic hydroxy compound other than the tetrahydroxy compound of formula (II) can be produced by allowing the aromatic hydroxy compound to react with an o-quinonediazide sulfonyl halide, such as 1,2-naphthoquinonediazide-4-sulfonyl chloride, 1,2-naphthoquinonediazide-5-sulfonyl chloride and 1,2-benzoquinonediazide-4-sulfonyl chloride, in the presence of a base, such as a base exemplified above as the one usable for producing a compound of formula (I), in a solvent such as the solvent above exemplified as the one usable for producing a compound of formula (I).

The aromatic hydroxy compound of formula (III) can be produced by allowing a compound represented by the following formula (IIIa):

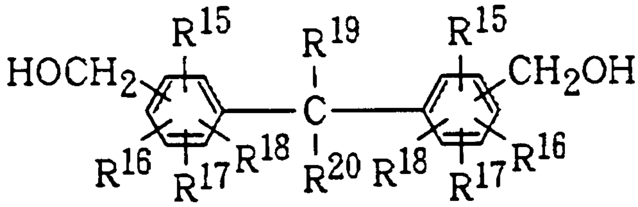

(IIIa)

wherein $R^{15}$, $R^{16}$, $R^{17}$, $R^{18}$, $R^{19}$ and $R^{20}$ are as defined above, to react with a compound represented by the following formula (IIIb):

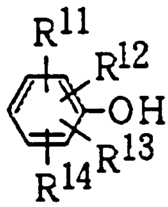

(IIIb)

wherein $R^{11}$, $R^{12}$, $R^{13}$ and $R^{14}$ are as defined above in the presence of an acid catalyst such as hydrochloric acid and p-toluene sulfonic acid.

Any ratio of the o-quinonediazide sulfonic acid ester of an aromatic hydroxy compound other than the tetrahydroxy compound of formula (II) to the compound of formula (I) can be adopted as long as the object of the present invention can be attained.

An alkali soluble novolac resin which is another ingredient of a positive resist composition can be produced through a condensation reaction of a compound having at least one phenolic hydroxy group with an aldehyde in the presence of an acid catalyst. Examples of the compound having at least one phenolic hydroxy group include o-cresol, m-cresol, p-cresol, 2,5-xylenol, 3,5-xylenol, 3,4-xylenol, 2,3,5-trimethylphenol, 2-isopropyl-5-methylphenol, 2-cyclohexyl-5-methylphenol and 2-t-butyl-5-methylphenol. The compound having at least one phenolic hydroxy group can be used singly or as a mixture of two or more. Examples of the aldehyde, other raw material for the alkali soluble novolac resin, include aliphatic aldehydes such as formaldehyde, acetaldehyde and glyoxal, and aromatic aldehydes

such as benzaldehyde, salicylaldehyde and terephthalaldehyde. From the industrial view, formaldehyde is preferred, because formaldehyde is industrially mass-produced as 37 % by weight of aqueous solution. The aldehyde can be used singly or as a mixture of two or more.

As the acid catalyst, inorganic acids such as hydrochloric acid, sulfuric acid and phosphoric acid, organic acids such as oxalic acid, acetic acid and p-toluenesulfonic acid, and divalent metal salts such as zinc acetate can be used. The condensation reaction can be carried out according to a conventional method. For example, it is carried out at a reaction temperature of 60 to 120 °C for 2 to 30 hours. The condensation reaction can be carried out in the absence or in the presence of a solvent.

Preferably, in order to reduce undeveloped residue, the alkali soluble novolac resin is processed, by a method such as fractionation, into a novolac resin in which the areal ratio of the GPC pattern of a range in that the molecular weight as converted to polystyrene is not higher than 900 is 25% or less, and more preferably 20% or less, based on the total pattern area excluding the pattern area of unreacted phenol compounds, as measured by using an UV 254 nm detector. The fractionation can be carried out, for example, through a method in which the alkali soluble novolac resin obtained through the condensation reaction is dissolved in a good solvent, and then the solution thus obtained is poured into water to precipitate the high molecular weight part of the alkali soluble novolac resin, or the solution is mixed with a poor solvent, such as pentane, hexane and heptane and then the mixed solution is separated into two phases. Examples of the good solvent include alcohols, such as methanol and ethanol; ketones, such as acetone and methylethylketone; ethyleneglycol ethers, such as ethylcellosolve; ethyleneglycol etheresters, such as ethylcellosolve acetate; and ethers, such as tetrahydrofuran.

The positive resist composition of the present invention may contain an alkali-soluble polyhydric phenol having a molecular weight of 900 or lower in addition to the above-mentioned alkali-soluble novolac resin and photosensitizer. As preferred examples of the alkali-soluble polyhydric phenol,

1,1-bis(4-hydroxyphenyl) cyclohexane;

a compound represented by the general formula (VII):

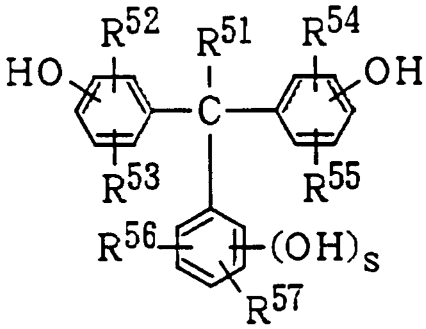

(VII)

wherein $R^{51}$ represents hydrogen or alkyl having 6 or less carbon atoms, and $R^{52}$, $R^{53}$, $R^{54}$, $R^{55}$, $R^{56}$ and $R^{57}$ each independently represent hydrogen, alkyl having 6 or less carbon atoms or cycloalkyl having 6 or less carbon atoms;

a compound represented by the following formula (VIII):

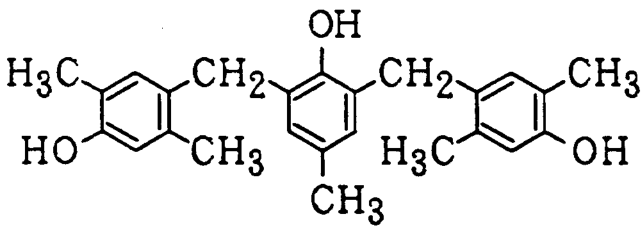

(VIII)

and compound (A) can be mentioned.

The alkali-soluble polyhydric phenol having a molecular weight of 900 or lower is usually used in an amount of 0-40% by weight, preferably 3-40% by weight, based on the total solid component of the positive resist composition.

The photosensitizer is preferably used in an amount of 10-50% by weight based on the total solid component of the positive resist composition.

Resist solutions can be formulated by mixing and dissolving a photosensitizer comprising a compound of formula (I) and another o-quinonediazide sulfonic acid ester of an aromatic hydroxy compound, if used; an alkali-soluble novolac resin; and other ingredients such as an alkali-soluble polyhydric phenol having a molecular weight of 900 or lower in a solvent.

As the solvent used in the preparation of the resist solution, solvents vaporizable at an appropriate rate and capable of giving a uniform and smooth coating film after evaporation can be used. Examples of such solvent include glycol etheresters, such as propylene glycol monomethyl ether acetate, ethyl cellosolve acetate and methyl cellosolve acetate; esters, such as n-amyl acetate, ethyl acetate, ethyl lactate, ethyl pyruvate and γ-butyrolactone; ketones, such as 2-heptanone; the solvents mentioned in JP-A-2-220056; the solvents mentioned in JP-A-4-362645; and the solvents mentioned in JP-A-4-367863. These solvents are used either independently or as a mixture of two or more. Considering the vaporization rate of solvents and the smoothness of the resist film to be obtained, the mixing ratio of the solid content to the solvent is determined.

As mentioned above, compound (A) can be used as a raw material for producing the compound of formula (I) or can be used as an ingredient of a resist composition comprising the compound of formula (I). As the ingredient, i.e. an alkali soluble polyhydric phenol compound, compound (A) is added as it is to the resist composition.

Compound (A) can also be used as an ingredient of a resist composition not comprising the compound of formula (I) but further comprising other photosensitizers and an alkali soluble novolac resin. This resist composition is also sensitive to radiations such as ultraviolet ray and far ultraviolet ray including excimer laser, and is usable in a minute producing process for a semiconductor. The alkali-soluble novolac resin used in the resist composition is not limited and the above-mentioned alkali-soluble novolac resin used in the positive resist composition comprising the compound of formula (I) can be used. The photosensitizer may be an o-quinonediazide sulfonic acid ester of an aromatic hydroxy compound other than the compound of formula (II). Examples of the aromatic hydroxy compound include polyhydroxy benzophenones, such as trihydroxy benzophenone, tetrahydroxy benzophenone and pentahydroxy benzophenone, polyhydroxy flavanes, such as 2,4,4-trimethyl-2',4',7-trihydroxy flavan, 2,4,4-trimethyl-2',3',4',7,8-pentahydroxy flavan, 6-hydroxy-4a-(2,4-dihydroxyphenyl)-1,2,3,4,4a,9a-hexahydroxanethene-9-spiro-1'-cyclohexane, and compounds having 2 or more phenolic hydroxy groups and having 2-4 benzene rings connected to each other through one or more aliphatic hydrocarbon connecting groups. More concrete examples of the photosensitizer are described in, for example, USP-5,124,228 (JP-A-2-32352), EP-A-363,978 (JP-A-2-103543), USP-5,290,656 (JP-A-2-269351), USP-5,283,155 (JP-A-3-185447), USP-5,188,920 (JP-A-4-50851), EP-A-505,987 (JP-A-4-295472), EP-A-570,884 (JP-A-5-323597) and EP-A-573,056 (JP-A-6-167805).

In the resist composition not comprising the compound of formula (I), the amount of compound (A) is preferably 3-40% by weight based on the total solid component of the resist composition, and the amount of the photosensitizer is preferably 10-50% by weight based on the total solid component of the resist composition.

The above mentioned solvents usable for the positive resist composition comprising the compound of formula (I) can also be used in the positive resist composition not comprising the compound of formula (I) but containing compound (A).

The positive resist composition may further comprise a small amount of dye or other resin, if required.

The present invention is explained in more detail with reference to the following examples. In the examples, part means part by weight.

Example 1

Into a 5 l three necked flask, 1009g of pyrogallol, 30.43g of p-toluenesulfonic acid and 1009g of water were charged. Then, 761g of 4-hydroxymethyl-2,5-dimethylphenol was added thereto dividedly over 1.5 hours while keeping the reaction temperature at 35°C. Thereafter, the reaction mixture was kept at 35°C for 1.5 more hours and was then cooled to room temperature to precipitate a crystalline product. The crystalline product obtained by filtration was added to and dissolved in a mixed solvent consisting of 400g of toluene and 2400g of ethylacetate. Then, the solution was washed with ion-exchanged water to remove metal contained in the solution, and was concentrated and filtered. The product thus obtained was dried and then further dried under reduced pressure for 24 hours to obtain 450g of 1,2,3-trihydroxy-4-(4-hydroxy-2,5-dimethylbenzyl)benzene.

Mass Spectrometry: MS 260
$^1$HNMR (acetone) δ ppm:
2.11(s, 6H); 3.74(s, 2H); 6.16(d, J=8.5Hz, 1H); 6.27(d, J=8.5Hz, 1H); 6.62(s, 1H); 6.80(s, 1H); 7.54(brs, 4H)

Example 2

Into a 5 l three necked flask, 1009g of pyrogallol, 30.43g of p-toluenesulfonic acid and 1009g of water were charged. Then, 761g of 4-hydroxymethyl-2,5-dimethylphenol was added thereto dividedly over 1.5 hours while keeping the reaction temperature at 35°C, and then the reaction temperature was kept at 35°C for 1.5 more hours. Thereafter, the reaction mixture was cooled to room temperature to precipitate a crystalline product. The crystalline product obtained by filtration was added to and completely dissolved in 1160g of methanol at a temperature of 50 °C. Then, to the solution, 1740g of ion-exchanged water was added and the mixture was cooled to 20 °C to precipitate a crystalline product which was then filtered. The filter cake was dried and further dried under a reduced pressure for 24 hours to obtain 500g of 1,2,3-trihydroxy-4-(4-hydroxy-2,5-dimethylbenzyl)benzene (compound A).

By mass spectrometry and NMR analysis, the compound obtained in this Example is identified as the same compound as that of Example 1.

According to the same method as in Example 1 or 2, except that 4-hydroxymethyl-2,5-dimethylphenol was replaced with other 4-hydroxymethylphenol or 4-alkenylphenol, tetrahydroxy compounds of the above mentioned formula (c) or (d) can be obtained.

Example 3 (Reference)

Into a mixture of 4.0g of 1,2,3-trihydroxy-4-(4-hydroxybenzyl)benzene, 18.5g of 1,2-naphthoquinonediazide-5-sulfonyl chloride and 113g of 1,4-dioxane, 8.36g of triethylamine was dropwise added at 20 - 30 °C over 1 hour. After completion of the addition, the mixture was stirred at 30°C for 3 hours. Thereto, 2.04g of acetic acid was added and then the mixture was stirred at 30 °C for 1 hour. The reaction mixture thus obtained was filtered and the obtained filter cake was washed with 1,4-dioxane. The filtrate and the 1,4-dioxane having been used for washing the filter cake were collected and added to a mixture consisting of 5.24g of acetic acid and 524g of ion exchanged water, and the mixture was stirred for 1 hour. Thereafter, the precipitated crystalline product was filtered, and the filter cake was washed with water and dried at 40 °C to obtain Sensitizer A, main content of which is diphenylmethane-2,3,4,4'-tetrayl tetrakis(6-diazo-5,6-dihydro-5-oxo-1-naphthalenesulfonate).

Mass Spectrometry: MS 1160

Example 4

Into a mixture of 4.0g of 1,2,3-trihydroxy-4-(4-hydroxy-2,5-dimethylbenzyl)benzene (compound A, compound of formula(b)), 16.5g of 1,2-naphthoquinonediazide-5-sulfonyl chloride and 103g of 1,4-dioxane, 7.48g of triethylamine was dropwise added at 20 - 30 °C over 1 hour. After completion of the addition, the mixture was stirred at 30°C for 3 hours. Thereto, 1.86g of acetic acid was added and the mixture was stirred at 30°C for 1 hour. The reaction mixture thus obtained was filtered and the obtained filter cake was washed with 1,4-dioxane. The filtrate and the 1,4-dioxane having been used for washing the filter cake were collected and added to a mixture consisting of 3.58g of acetic acid and 358g of ion exchanged water, and the mixture was stirred for 1 hour. Thereafter, the precipitated crystalline product was filtered, and the filter cake was washed with water and dried at 40 °C to obtain Sensitizer B, main content of which is 2',5'-dimethyldiphenylmethane-2,3,4,4'-tetrayl tetrakis(6-diazo-5,6-dihydro-5-oxo-1-naphthalenesulfonate).

Mass Spectrometry: MS 1188

Example 5

Into a mixture of 4.0g of 1,2,3-trihydroxy-4-(4-hydroxy-2,3,6-trimethylbenzyl)benzene (compound of formula(c)), 15.7g of 1,2-naphthoquinonediazide-5-sulfonyl chloride and 98g of 1,4-dioxane, 7.08g of triethylamine was dropwise added at 20 - 30 °C over 1 hour. After completion of the addition, the mixture was stirred at 30°C for 12 hours. Thereto, 1.76g of acetic acid was added and then the mixture was stirred at that temperature for 1 hour. The reaction mixture thus obtained was filtered and the obtained filter cake was washed with 1,4-dioxane. The filtrate and the 1,4-dioxane having been used for washing the filter cake were collected and added to a mixture consisting of 3.43g of acetic acid and 342g of ion exchanged water, and the mixture was stirred for 1 hour. Thereafter, the precipitated crystalline product was filtered, and the filter cake was washed with water and dried at 40 °C to obtain Sensitizer C, main content of which is 2',3',6'-trimethyldiphenylmethane-2,3,4,4'-tetrayl tetrakis(6-diazo-5,6-dihydro-5-oxo-1-naphthalenesulfonate).

Mass Spectrometry: MS 1202

Example 6

Into a mixture of 4.0g of 1,2,3-trihydroxy-4-(4-hydroxy-2,3,5-trimethylbenzyl)benzene (compound of formula(d)), 15.7g of 1,2-naphthoquinonediazide-5-sulfonyl chloride and 98g of 1,4-dioxane, 7.08g of triethylamine was dropwise added at 20 - 30 °C over 1 hour. After completion of the addition, the mixture was stirred at 30°C for 3 hours. Thereto, 1.76g of acetic acid was added and then the mixture was stirred at that temperature for 1 hour. The reaction mixture thus obtained was filtered and the obtained filter cake was washed with 1,4-dioxane. The filtrate and the 1,4-dioxane having been used for washing the filter cake were collected and added to a mixture consisting of 3.43g of acetic acid and 342g of ion exchanged water, and the mixture was stirred for 1 hour. Thereafter, the precipitated crystalline product was filtered, and the filter cake was washed with water and dried at 40°C to obtain Sensitizer D, main content of which is 2',3',5'-trimethyldiphenylmethane-2,3,4,4'-tetrayl tetrakis(6-diazo-5,6-dihydro-5-oxo-1-naphthalenesulfonate).

Mass Spectrometry: MS 1202

Example 7

Into a mixture of 4.0g of 1,2,3-trihydroxy-4-(4-hydroxy-2,5-dimethylbenzyl)benzene (compound A, compound of formula(b)), 13.4g of 1,2-naphthoquinonediazide-5-sulfonyl chloride and 87g of 1,4-dioxane, 6.08g of triethylamine was dropwise added at 20 - 30°C over 1 hour. After completion of the addition, the mixture was stirred at 30°C for 3 hours. Thereto, 1.51g of acetic acid was added and then the mixture was stirred at that temperature for 1 hour. The reaction mixture thus obtained was filtered and the obtained filter cake was washed with 1,4-dioxane. The filtrate and the 1,4-dioxane having been used for washing the filter cake were collected and added to a mixture consisting of 5.24g of acetic acid and 524g of ion exchanged water, and the mixture was stirred for 1 hour. Thereafter, the precipitated crystaline product was filtered, and the filter cake was washed with water and dried at 40°C to obtain Sensitizer E, main content of which is 1,2-naphthoquinonediazide-5-sulfonic acid triester of 1,2,3-trihydroxy-4-(4-hydroxy-2,5-dimethylbenzyl)benzene and which further contains monoester, diester and tetraester thereof.

Mass Spectrum of this compound has the main peak at 956 and also has relatively large peaks at 1188 and 724.

Example 8 (Reference)

Example 4 was repeated, except that 4.0g of 1,2,3-trihydroxy-4-(4-hydroxy-2,5-dimethylbenzyl)benzene was replaced with 4.0g of 1,2,3-trihydroxy-4-(2-hydroxy-3,5-dimethylbenzyl)benzene to obtain Sensitizer I, main content of which is 3',5' -dimethyl diphenyl methane-2,2',3,4-tetrayl tetrakis(6-diazo-5,6-dihydro-5-oxo-1-naphthalene sulfonate).

Mass Spectrometry: MS 1188

Example 9

Into a 100 ml four necked flask, 2.6g of 1,2,3-trihydroxy-4-(4-hydroxy-2,5-dimethylbenzyl)benzene (compound A, compound of formula(b)), 10.75g of 1,2-naphthoquinone diazide-5-sulfonic acid chloride and 66.75g of 1,4-dioxane were charged and completely dissolved. Into the mixture, 4.86g of triethylamine was dropwise added at 20 - 30°C over 1 hour. After completion of the addition, the mixture was stirred at 30°C for 3 hours. Thereto, 1.20g of acetic acid was added and then the mixture was stirred at that temperature for 1 hour. The reaction mixture thus obtained was filtered and the obtained filter cake was washed with 10.75g of 1,4-dioxane. The filtrate and the 1,4-dioxane having been used for washing the filter cake were collected and added to a mixture consisting of 2.71g of acetic acid and 271g of ion exchanged water, and the mixture was stirred for 1 hour. Thereafter, the precipitated crystalline product was filtered. The filter cake thus obtained was added to 300g of ion exchanged water, and, then, the mixture was stirred and filtered. After this water wash process was repeated three more times, the filter cake was dried at 40°C to obtain Sensitizer F, main content of which is 2',5'-dimethyl diphenyl methane-2,3,4,4'-tetrayl tetrakis(6-diazo-5,6-dihydro-5-oxo-1-naphthalene sulfonate).

Mass Spectrometry: MS 1188

$^1$HNMR (dimethyl sulfoxide) $\delta$ ppm:

1.80(s, 3H); 2.00(s, 3H); 3.70(s, 2H); 6.56(s, 1H); 6.58(d, J=10Hz, 1H); 6.69(d, J=10Hz, 1H); 6.80(s, 1H); 7.00(m, 3H); 7.35(m, 1H); 7.60(m, 8H); 7.95(d, J=6Hz, 1H); 8.01(d, J=6Hz, 1H); 8.17(d, J=6Hz, 1H); 8.24(d, 1H); 8.34(d,

J=7.5Hz, 1H); 8.52(m, 2H); 8.60(d, J=7.5Hz, 1H)

Reference example 1

Into a 1000ml four necked flask, 148.5g of m-cresol, 121.5g of p-cresol, 252g of methylisobutyl ketone, 37.0g of 10% aqueous oxalic acid solution and 84.8g of 90% aqueous aceticacid solution were charged, and thereto, 129.5g of 37% formalin was added dropwise over 40 minutes while being heated with an oil bath at a temperature of 100 °C. Then, the reaction mixture was kept for 15 more hours to continue the reaction. Then, the reaction mixture was washed with water and dehydrized to obtain 466g of methylisobutylketone solution containing 42.3% of a novolac resin. As measured by GPC, the product thus obtained had a polystyrene-converted weight average molecular weight of 4300.

Into a 5 l separation flask equipped with a drain pipe at its bottom, 450g of the solution thus obtained was charged. Thereto, 909.6g of methyl isobutyl ketone and 996.1g of n-heptane were further added. The mixture was stirred at 60°C for 30 minutes and then left to stand until it separated into two layers. To the lower layer obtained by phase separation, 380g of 2-heptanone was added, after which the methyl isobutyl ketone and n-heptane were removed by means of an evaporator to obtain a solution of novolac resin in 2-heptanone. As-measured by GPC, the product thus obtained had a polystyrene-converted weight average molecular weight of 9000, and the areal ratio of the range in which the molecular weight as converted to polystyrene was 900 or less to the total pattern area was 14%.

Reference example 2

By a condensation reaction of m-cresol, p-cresol, 2,5-xylenol, salicylic aldehyde and formaldehyde in a molar ratio of 3/2/5/1/7, respectively, a novolac resin was obtained. It has a polystyrene-converted weight average molecular weight of 8000 as measured by GPC, and the areal ratio of the range in which the molecular weight as converted to polystyrene was 900 or less to the total pattern area is 20%.

Reference example 3

By condensation reactions between 1 part by mole of 4,4'-methylenebis[2-(4-hydroxybenzyl)-3,6-dimethylphenol] and 2 parts by moles of 1,2-naphthoquinonediazide-5-sulfonyl chloride, Sensitizer X and Sensitizer X' were obtained.

Example 10

15 Parts, calculated as the solid content, of novolac resin solution in 2-heptanone obtained in Reference example 1, 5 parts of Sensitizer X obtained in Reference example 3, 1 part of a condensate of 6-hydroxy-4a-(2,4-dihydroxyphenyl)-1,2,3,4,4a,9a-hexahydroxanethene-9-spiro-1'- cyclohexane having a structure of the following formula:

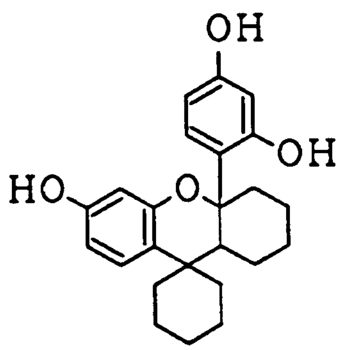

and 1,2-naphthoquinonediazide-5-sulfonyl chloride in an mole ratio of 1:3, respectively, and 3.9 parts of compound A were added to and dissolved in 2-heptanone so that the total amount of the solution become 50 parts. The solution thus obtained was filtered through a filter made from fluorine resin and having a pore size of 0.2 µm to prepare a resist solution. A silicon wafer washed in a conventional manner was coated with the resist solution thus prepared by means of a spin coater so that the thickness after being baked became 1.1 µm and it was baked on a hot plate at 90 °C for one minute. Subsequently, the wafer was exposed to light by using a reduction projection exposing machine having an exposure wavelength of 365 nm (i-line) (NSR1755i7A, NA=0.5, manufactured by Nikon Corp.) while stepwise changing the amount of exposure. Thereafter, the wafer was baked on a hot plate at 110 °C for one minute. Then, it was developed for one minute with SOPD (alkaline developing solution; product of Sumitomo Chemical Co., Ltd.) to obtain a positive pattern.

The positive pattern was evaluated according to methods mentioned below and results are shown in Table 1.

Table 1

| | |
|---|---|
| Effective sensitivity | 400 msec |
| Resolution | 0.35 μm |
| Profile | 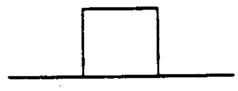 |
| Depth of focus : | 1.5 μm |
| γ-value : | 6.18 |
| Scum : | Not found |

Examples 11-15

10.43 parts of novolac resin obtained in Reference example 2, 3.9 parts of 4,4'-(2-hydroxybenzylidene)di-2,6-xylenol and a Sensitizer shown in Table 2 were dissolved in a mixed solvent consisting of 2-heptanone and γ-butyrolactone in an weight ratio of 95:5, respectively. The solution thus obtained was filtered through a filter having a fine pore size to prepare a resist solution. The amount of the solvent was adjusted so that the thickness of the coated resist film after being baked became 1.1 μm.

A silicon wafer washed in a conventional manner was coated with the resist solution by means of a spin coater and it was baked on a hot plate at 90 °C for one minute. Subsequently, the wafer was exposed to light by using a reduction projection exposing machine having an exposure wavelength of 365 nm (i-line) (NSR 1755i 7A, NA=0.5, manufactured by Nikon Corp.) while stepwise changing the amount of exposure. Thereafter, the wafer was baked on a hot plate at 110 °C for one minute. Then, it was developed for one minute with SOPD (alkaline developing solution; product of Sumitomo Chemical Co., Ltd.) to obtain a positive pattern.

The positive pattern was evaluated according to methods mentioned below and results are shown in Table 2.

Table 2

| Exampl No. | Formulation of resist | Properties of resist | | | | | |
|---|---|---|---|---|---|---|---|
| | Photo-sensitizer | Sensi-tivity | Resolu-tion | Profile | Heat resist-ance | $\gamma$-value | Scum |
| 11 (Reference) | A : 1 part<br>X' : 5 parts | 170 msec | 0.35 μm | 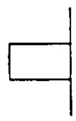 | 4 | 3.98 | Not found |
| 12 | B : 1 part<br>X' : 5 parts | 172 msec | 0.35 μm | 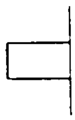 | 4 | 4.70 | Not found |
| 13 | C : 1 part<br>X' : 5 parts | 166 msec | 0.35 μm | 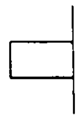 | 4 | 4.51 | Not found |
| 14 | D : 1 part<br>X' : 5 parts | 175 msec | 0.35 μm | 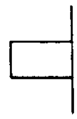 | 4 | 4.10 | Not found |
| 15 | E : 3.5 parts | 76 msec | 0.40 μm | 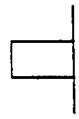 | 4 | 4.58 | Not found |

Sensitivity : expressed in exposure amount (time) at which the film thickness become zero on a graph which was

obtained by plotting the exposure amount against the retained film thickness.

Effective sensitivity (in Example 10) : expressed in exposure amount (time) giving 1:1 line-and-space of 0.50 µm.

Resolution : evaluated by measuring, with a scanning electron microscope, the dimension of the minimum line-and-space pattern which could be resolved without film thickness decrease at an exposure amount giving 1:1 line-and-space(0.50 µm in Example 10, 0.40 µm in Examples 11-15).

Profile : evaluated by observing, with a scanning electron microscope, the cross-sectional shape of a 0.45 µm line-and-space pattern at the effective sensitivity.

The depth of focus : determined by observing, with a scanning electron microscope, a degree of focus shifting where a 0.40 µm line-and-space pattern could be resolved at the effective sensitivity without film thickness decrease.

Heat resistance : evaluated by observing, with a scanning electron microscope, the extent of sagging of the resist pattern after being heated on a hot plate at 124 °C for 5 minutes. Heat resistance is expressed by 5 grade expression in which 5 is best and 1 is worst.

γ-value : expressed in term of tan θ. The θ is obtained by plotting a normalized film thickness (=the retained film thickness/ the original film thickness) against a logarithm of the exposure amount and calculating the inclination of the plotted line.

Scum : evaluated by observing, with a scanning electron microscope, undeveloped residue in the positive pattern.

The compound of the formula (I) is useful as a photosensitizer for a positive resist composition. The positive resist composition comprising the compound of the formula (I) is excellent in the balance among properties such as sensitivity, resolution, γ-value, profile and heat resistance, and free from scum.

An o-quinonediazide sulfonic acid ester of compound A can be used as a photosensitizer for a resist composition. Compound A can also be used as an alkali-soluble ingredient of a resist composition. Compound A can be produced easily in high purity. A positive resist composition comprising compound A is excellent in the balance among properties such as sensitivity, resolution, γ-value and profile, and free from scum. Therefore, it is very appropriate for minute production processes of semiconductor.

## Claims

1. A compound represented by the general formula (I):

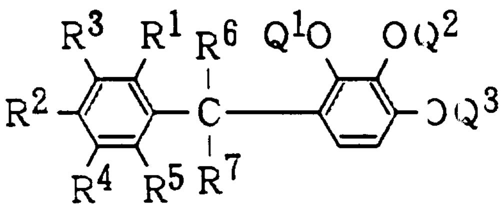

(I)

wherein $R^2$ represents $-OQ^4$ and $R^1$, $R^3$, $R^4$ and $R^5$ each independently represent hydrogen or alkyl having 6 or less carbon atoms, provided that at least one of $R^1$ and $R^5$ is alkyl having 6 or less carbon atoms;

$R^6$ and $R^7$ each independently represent hydrogen, alkyl having 6 or less carbon atoms or alkenyl having 6 or less carbon atoms, or $R^6$ and $R^7$ form a cycloalkane ring having 6 or less carbon atoms together with the carbon atom to which they are attached; and
$Q^1$, $Q^2$, $Q^3$ and $Q^4$ each independently represent hydrogen or an o-quinonediazide sulfonyl group, provided that at least one of $Q^1$, $Q^2$, $Q^3$ and $Q^4$ is an o-quinonediazide sulfonyl group.

2. A compound according to claim 1 wherein both of $R^3$ and $R^5$ are hydrogen, and both of $R^1$ and $R^4$ are methyl.

**3.** A compound according to claim 1 wherein all of $Q^1$, $Q^2$, $Q^3$ and $Q^4$ are o-quinonediazide sulfonyl groups.

**4.** 2',5'-Dimethyldiphenyl-2,3,4,4'-tetrayl tetrakis(6-diazo-5,6-dihydro-5-oxo-1-naphthalene sulfonate).

**5.** A process for producing a compound according to any one of Claims 1 to 4 which comprises allowing a tetrahydroxy compound represented by the formula (II):

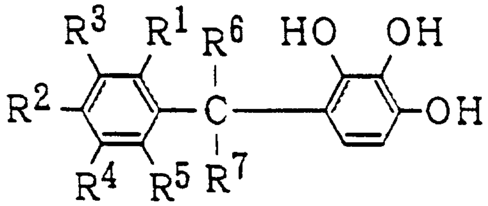

(II)

wherein $R^2$ represents hydroxy, and $R^1$ and $R^3$ are as defined in claim 1 to react with an o-quinonediazide sulfonyl halide in the presence of a base.

**6.** A photosensitizer comprising a compound according to any one of claims 1 to 4.

**7.** A positive resist composition comprising a compound according to any one of claims 1 to 4 and an alkali-soluble novolac resin.

**8.** A positive resist composition according to claim 7 wherein the alkali soluble novolac resin has a GPC pattern in which the areal ratio of a range in that a molecular weight as converted to polystyrene is not higher than 900 is 25% or less, based on the total pattern area excluding the pattern area of unreacted phenol compounds, as measured by using an UV 254 nm detector.

**9.** A positive resist composition according to claim 7 which further comprises an alkali-soluble polyhydric phenol compound having a molecular weight of 900 or lower.

**10.** 1,2,3-Trihydroxy-4-(4-hydroxy-2,5-dimethylbenzyl) benzene (formula (A)).

**11.** A process for producing 1,2,3-trihydroxy-4-(4-hydroxy-2,5-dimethylbenzyl)benzene which comprises allowing 4-hydroxymethyl-2,5-dimethylphenol to react with pyrogallol.

**12.** A process according to claim 11 wherein the molar ratio of 4-hydroxymethyl-2,5-dimethylphenol to pyrogallol is 1:1-3.

**13.** A process according to claim 11 wherein 0.25-5 parts by weight of water based on 1 part by weight of pyrogallol is used as a solvent.

**14.** A process according to claim 11 wherein the reaction product is subjected to crystallization from a solution in an aromatic solvent or from an aqueous solution of alcohol.

**15.** A positive resist composition comprising 1,2,3-trihydroxy-4-(4-hydroxy-2,5-dimethylbenzyl)benzene.

**16.** A positive resist composition according to claim 15 which further comprises an alkali-soluble novolac resin and an o-quinonediazide sulfonic acid ester.

**Patentansprüche**

**1.** Verbindung der allgemeinen Formel (I):

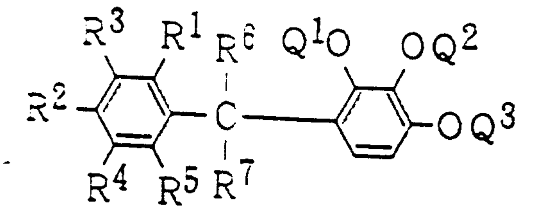

(I)

in welcher $R^2$ den Rest $-OQ^4$ darstellt und $R^1$, $R^3$, $R^4$ und $R^5$ jeweils unabhängig Wasserstoff oder ein Alkylrest mit 6 oder weniger Kohlenstoffatomen sind, mit der Maßgabe, daß mindestens einer der Reste $R^1$ und $R^5$ ein Alkylrest mit 6 oder weniger Kohlenstoffatomen ist;

$R^6$ und $R^7$ jeweils unabhängig Wasserstoff, einen Alkylrest mit 6 oder weniger Kohlenstoffatomen oder einen Alkenylrest mit 6 oder weniger Kohlenstoffatomen darstellen, oder $R^6$ und $R^7$ zusammen mit dem Kohlenstoffatom, an welches sie gebunden sind, einen Cycloalkanring mit 6 oder weniger Kohlenstoffatomen bilden; und 4

$Q^1$, $Q^2$, $Q^3$ und $Q^4$ jeweils unabhängig Wasserstoff oder eine o-Chinondiazidsulfonylgruppe sind, mit der Maßgabe, daß mindestens einer der Reste $Q^1$ , $Q^2$, $Q^3$ und $Q^4$ eine o-Chinondiazidsulfonylgruppe ist.

**2.** Verbindung gemäß Anspruch 1, in welcher beide Reste $R^3$ und $R^5$ Wasserstoffatome sind und beide Reste $R^1$ und $R^4$ Methylgruppen sind.

**3.** Verbindung gemäß Anspruch 1, in welcher alle Reste $Q^1$ ,$Q^2$, $Q^3$ und $Q^4$ o-Chinondiazidsulfonylgruppen sind.

**4.** 2'5'-Dimethyldiphenyl-2,3,4,4'-tetrayltetrakis(6-diazo-5,6-dihydro-5-oxo-1-naphthalinsulfonat).

**5.** Verfahren zur Herstellung einer Verbindung gemäß einem der Ansprüche 1 bis 4, umfassend die Umsetzung einer Tetrahydroxyverbindung der Formel (II)

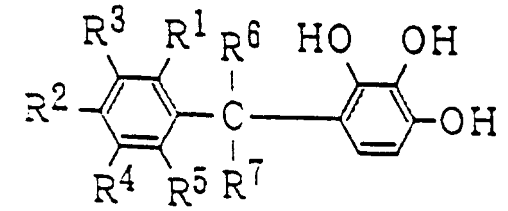

(II)

in welcher $R^2$ eine Hydroxygruppe ist und $R^1$ und $R^3$ bis $R^7$ wie in Anspruch 1 definiert sind, mit einem o-Chinondiazidsulfonylhalogenid in Gegenwart einer Base.

**6.** Photosensibilisator, umfassend eine Verbindung gemäß einem der Ansprüche 1 bis 4.

**7.** Positiv arbeitende Resistzusammensetzung, umfassend eine Verbindung gemäß einem der Ansprüche 1 bis 4 und ein alkalilösliches Novolakharz.

**8.** Positiv arbeitende Resistzusammensetzung gemäß Anspruch 7, wobei das alkalilösliche Novolakharz ein GPC-Muster besitzt, in welchem das Flächenverhältnis eines Bereichs, in welchem das auf Polystyrol umgerechnete Molekulargewicht nicht größer als 900 ist, 25 % oder weniger beträgt, bezogen auf den Gesamtmusterbereich ohne den Musterbereich von nicht umgesetzten Phenolverbindungen, gemessen unter Verwendung eines UV-Detektors bei 254 nm.

**9.** Positiv arbeitende Resistzusammensetzung gemäß Anspruch 7, welche ferner eine alkalilösliche, mehrwertige Phenolverbindung mit einem Molekulargewicht von 900 oder weniger umfaßt.

**10.** 1,2,3-Trihydroxy-4-(4-hydroxy-2,5-dimethylbenzyl)benzol (Formel (A)).

**11.** Verfahren zur Herstellung von 1,2,3-Trihydroxy-4-(4-hydroxy-2,5-dimethylbenzyl)benzol, umfassend die Umsetzung von 4-Hydroxymethyl-2,5-dimethylphenol mit Pyrogallol.

**12.** Verfahren gemäß Anspruch 11, in welchem das Molverhältnis von 4-Hydroxymethyl-2,5-dimethylphenol zu Pyrogallol 1 : 1-3 beträgt.

**13.** Verfahren gemäß Anspruch 11, in welchem 0,25 bis 5 Gewichtsteile Wasser, bezogen auf ein Gewichtsteil Pyrogallol, als Lösungsmittel verwendet wird.

**14.** Verfahren gemäß Anspruch 11, in welchem das Reaktionsprodukt einer Kristallisation aus einer Lösung in einem aromatischen Lösungsmittel oder aus einer wäßrigen Lösung eines Alkohols unterworfen wird.

**15.** Positiv arbeitende Resistzusammensetzung, umfassend 1,2,3-Trihydroxy-4-(4-hydroxy-2,5-dimethylbenzyl)benzol.

**16.** Positiv arbeitende Resistzusammensetzung gemäß Anspruch 15, welche ferner ein alkalilösliches Novolakharz und einen o-Chinondiazidsulfonsäureester umfaßt.

**Revendications**

**1.** Composé représenté par la formule générale (I) :

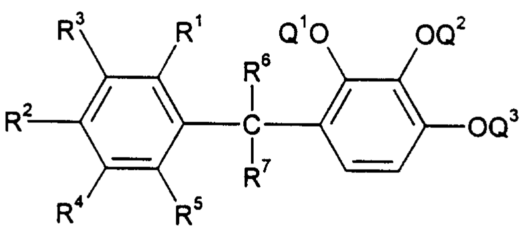

(I)

dans laquelle $R^2$ représente $-OQ^4$ et chacun de $R^1$, $R^3$, $R^4$ et $R^5$ représente indépendamment l'hydrogène ou un radical alkyle ayant 6 atomes de carbone ou moins, du moment qu'au moins l'un de $R^1$ et $R^5$ est un radical alkyle ayant 6 atomes de carbone ou moins ;

chacun de $R^6$ et $R^7$ représente indépendamment l'hydrogène, un radical alkyle ayant 6 atomes de carbone ou moins ou un radical alcényle ayant 6 atomes de carbone ou moins, ou bien $R^6$ et $R^7$ forment un cycle cycloalcane ayant 6 atomes de carbone ou moins conjointement avec l'atome de carbone auquel ils sont rattachés ; et chacun de $Q^1$, $Q^2$, $Q^3$ et $Q^4$ représente indépendamment l'hydrogène ou un groupe o-quinonediazidosulfonyle, du moment qu'au moins l'un de $Q^1$, $Q^2$, $Q^3$ et $Q^4$ est un groupe o-quinonediazidosulfonyle.

**2.** Composé selon la revendication 1, dans lequel $R^3$ et $R^5$ sont tous deux l'hydrogène, et $R^1$ et $R^4$ sont tous deux le radical méthyle.

**3.** Composé selon la revendication 1, dans lequel $Q^1$, $Q^2$, $Q^3$ et $Q^4$ sont tous des groupes o-quinonediazidosulfonyle.

**4.** Tétrakis(6-diazo-5,6-dihydro-5-oxo-1-naphtalènesulfonate) de 2',5'-diméthyldiphényl-2,3,4,4'-tétrayle.

**5.** Procédé pour produire un composé selon l'une quelconque des revendications 1 à 4, qui consiste à laisser un composé tétrahydroxylé représenté par la formule (II) :

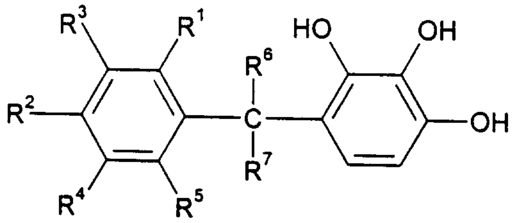

(II)

dans laquelle $R^2$ représente un radical hydroxy et $R^1$ et $R^3$ à $R^7$ sont tels que définis dans la revendication 1, réagir avec un halogénure de o-quinonediazidosulfonyle en présence d'une base.

**6.** Photosensibilisant comprenant un composé selon l'une quelconque des revendications 1 à 4.

**7.** Composition de réserve positive comprenant un composé selon l'une quelconque des revendications 1 à 4 et une résine novolaque soluble dans les alcalis.

**8.** Composition de réserve positive selon la revendication 7, dans laquelle la résine novolaque soluble dans les alcalis a un motif de CPG dans lequel le rapport d'aire d'une plage dans laquelle la masse moléculaire, convertie en polystyrène, n'est pas supérieure à 900, est de 25 % ou moins, sur la base de l'aire de motif totale à l'exclusion de l'aire de motif de composés phénoliques n'ayant pas réagi, telle que mesurée par utilisation d'un détecteur UV à 254 nm.

**9.** Composition de réserve positive selon la revendication 7, qui comprend en outre un composé phénolique polyvalent soluble dans les alcalis ayant une masse moléculaire de 900 ou moins.

**10.** 1,2,3-trihydroxy-4-(4-hydroxy-2,5-diméthylbenzyl)-benzène (formule (A)).

**11.** Procédé pour produire du 1,2,3-trihydroxy-4-(4-hydroxy-2,5-diméthylbenzyl)benzène, qui consiste à laisser du 4-hydroxyméthyl-2,5-diméthylphénol réagir avec du pyrogallol.

**12.** Procédé selon la revendication 11, dans lequel le rapport molaire du 4-hydroxyméthyl-2,5-diméthylphénol au pyrogallol est de 1/1-3.

**13.** Procédé selon la revendication 11, dans lequel on utilise, en tant que solvant, 0,25 à 5 parties en poids d'eau pour 1 partie en poids de pyrogallol.

**14.** Procédé selon la revendication 11, dans lequel le produit réactionnel est soumis à une cristallisation à partir d'une solution dans un solvant aromatique ou à partir d'une solution aqueuse d'alcool.

**15.** Composition de réserve positive comprenant du 1,2,3-trihydroxy-4-(4-hydroxy-2,5-diméthylbenzyl)benzène.

**16.** Composition de réserve positive selon la revendication 15, qui comprend en outre une résine novolaque soluble dans les alcalis et un ester d'acide o-quinonediazidosulfonique.